# EUROPEAN PATENT SPECIFICATION

(11) **EP 4 143 160 B1**
(45) Date of publication and mention of the grant of the patent: **01.11.2023**
(21) Application number: 21717900.1
(22) Date of filing: 14.04.2021
(51) Int. Cl.: C07C 319/20, C07C 323/59, C07C 323/25

(54) **PROCESS OF MAKING N,N'-DIACETYL-L-CYSTINE**
VERFAHREN ZUR HERSTELLUNG VON N,N'-DIACETYL-L-CYSTIN
PROCÉDÉ DE FABRICATION DE N,N-DIACÉTYL-L-CYSTINE

(30) Priority: 28.04.2020 EP 20171854
(43) Date of publication of application: 08.03.2023
(73) Proprietor: Unilever IP Holdings B.V., 3013 AL Rotterdam (NL); Unilever Global IP Limited, Wirral, Merseyside CH62 4ZD (GB)
(72) Inventor: ROSA, Jose Guillermo, Trumbull, Connecticut 06611 (US); HARICHIAN, Bijan, Trumbull, Connecticut 06611 (US)
(74) Representative: James, Helen Sarah
(86) International application number: PCT/EP2021/059650
(87) International publication number: WO 2021/219375

(56) References cited:
- Leonore Hollander: "THE RESOLUTION OF INACTIVE CYSTINE AND ISOLA- TION OF PURE DEXTROROTATORY CYSTINE", Journal of Biological Chemistry, 1 January 1931 (1931-01-01), pages 243-252, XP055727401, Retrieved from the Internet: URL:https://www.jbc.org/content/94/1/243.s hort [retrieved on 2020-09-03]
- SCOTT J. PYE ET AL: "Organic oxidations promoted in vortex driven thin films under continuous flow", RCS, GREEN CHEMISTRY, vol. 20, no. 1, 1 January 2018 (2018-01-01), pages 118-124, XP055725735, GB ISSN: 1463-9262, DOI: 10.1039/C7GC03352D cited in the application
- VANDANA RATHORE ET AL: "An Organodiselenide with Dual Mimic Function of Sulfhydryl Oxidases and Glutathione Peroxidases: Aerial Oxidation of Organothiols to Organodisulfides", ORGANIC LETTERS, vol. 20, no. 19, 24 September 2018 (2018-09-24), pages 6274-6278, XP055727251, US ISSN: 1523-7060, DOI: 10.1021/acs.orglett.8b02756
- VLADIMIR N. BELOV ET AL: "Syntheses of D-Labelled Oxidative Metabolites of Acrylamide and Acrylonitrile for the Quantification of Their Toxicities in Humans", EUROPEAN JOURNAL OF ORGANIC CHEMISTRY, vol. 2008, no. 26, 1 September 2008 (2008-09-01), pages 4417-4425, XP055727417, DE ISSN: 1434-193X, DOI: 10.1002/ejoc.200800291 cited in the application

## Description

### Field of the invention

The present invention is directed to an efficient method of making N,N'-diacetyl-L-Cystine ("NDAC").

### Background of the invention

Healthy look is a universal consumer need and personal care is an aspect of every-day life. Healthy looking skin can be described in terms of attributes of appearance (glow, radiance, evenness of hue, pigmentation spots), texture (smoothness, silkiness, lack of bumps and pores), and age (fine lines, wrinkles, elasticity, and sagging/laxity). While different classes of compounds claim cosmetic benefits on skin appearance, cysteine and cystine derivatives have not received much attention.

A number of cysteine/cystine derivatives, including β-substituted cysteine/cystines, cystine diamides, cystine dialkyl esters and N-alkanoylcysteines have potential therapeutic benefits, for example in kidney stone prevention (See , Zhu, et al., "Rational Design of Novel Crystal Growth Inhibitors for Treatment of Cystinuria Kidney Stones," 2013 ProQuest Dissertations and Theses; CrystEngComm, 2016, 18, 8587). However, even the simplest derivative of cystine, N,N'-diacetyl-L-cystine ("NDAC"), lacks industrial application, especially in the beauty and personal care field.

As a result of few known uses, NDAC is practically not available on a commercial scale. It has now been discovered that NDAC breaks down into cystine, which is reduced to cysteine in cells of an organism, i.e., intracellular formation of cysteine. Cysteine and its dimer Cystine are glutathione precursors. Glutathione (GSH) is a tripeptide that consists of glutamate, cysteine, and glycine. It is present in all mammalian tissues. It is the main antioxidant in the living body, which protects cells from oxidation by quenching or scavenging reactive oxygen species (ROS). Excessive ROS in skin caused by solar irradiation and pollutants are associated with clinical manifestations such as uneven skin tone. Glutathione's role in mitigating melanogenesis has been previously reported. With ageing and exposure to stresses, glutathione decreases; thus, it is highly desirable to increase glutathione in skin to provide benefits, such as even skin tone.

Compositions for potentiating intracellular glutathione production have been described. See e.g. Chiba et al. US Patent 7,740,831, Crum et al (USRE37934, USRE42645, WO2016/033183, and US20050271726); Mammone US Patent No. 6,149,925, and Perricone US 2006/0063718. Topical compositions and enhancing generation of glutathione in skin from its constituent amino acids (glutamate, cysteine, and/or glycine, i.e., glutathione precursors) for cellular uptake and synthesis of the GSH tripeptide was addressed in, e.g., Applicant's U.S. Published Patent Application Nos.: US20/9034, US20/16059, and US19/328631.

Applicants have now discovered that the performance of NDAC is comparable to that of cystine for potentiating glutathione synthesis within skin cells, but NDAC is more soluble, does not generate unpleasant sulfurous odor compared to cysteine thiol (SH) containing derivatives or other cystine derivatives, and can have better delivery to skin, thereby resulting in better performance. Proteases in the body can cleave (or hydrolyze) the N-acetyl bonds of NDAC to ultimately generate cystine, thus, NDAC can also serve as a controlled release alternate source of cystine.

Accordingly, there is a need for the NDAC material.

Examples of other cystine derivatives which are less advantageous than NDAC are N-Acetyl Cysteine ("NACys") which contains a thiol (SH) group and L-Cystine diethyl ester ("DEC") which does not contain an SH-group. NACys is a thiol drug used commonly as an expectorant (Cryst.Eng.Comm., 2016, 18, 8587, referred to as "NACe" therein). NACys provides an upleasant sulfurous odor, that is unacceptable in cosmetic products. Furthermore, the sulfurous odor (monitored as hydrogen sulfide or H₂S) is consistent with NACys decomposition which is also unacceptable in marketed cosmetic products. Similarly, DEC also generates a strong undesirable sulfurous odor and is not stable in formulated cosmetic products.

NDAC is an amide, i.e., an N,N'-diacetyl derivative of cystine. NDAC, for purposes of the present invention, has the following chemical structure:

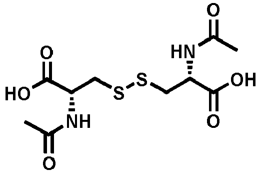

The molecular weight of NDAC = 324.4.

NDAC stereoisomers (referring to the stereoisomerism of the alpha-Carbon atom located between the Nitrogen atom and the Carbonyl group of the carboxylic acid moiety) of the present invention include R,R (L-cystine), R,S, S,R and S,S (D-cystine). Preferably, L stereoisomers are employed, and this is the most abundant and natural isomeric form found in nature.

NDAC is not readily commercially available but may be sourced on lab scale. NDAC may be synthesized directly from NACys as described in Vandana Rathore et al, Organic Letters, 20(19), 6274-6278; 2018 and Scott J. Pye et al, Green Chemistry, 20(1), 118-124; 2018:

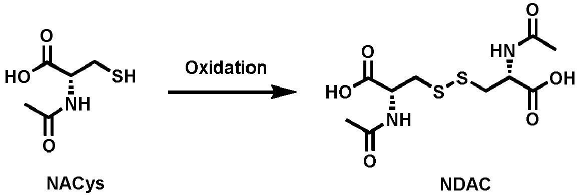

Such chemical processes involving oxidative dimerization of NACys, or other SH-containing raw materials, to generate NDAC suffer from significant disadvantages. First, any unreacted NACys is difficult to remove from the desired NDAC product and requires specialized purification methods such as chromatography using polar solvent systems. Second, such purification methods are impractical at very large scales (e.g. kg, tons). Third, even very small amounts of NACys impurities in the final NDAC product will render it unusable for cosmetic products as SH-containing impurities such as NACys will generate undesirable sulfurous odor when formulated. Fourth, during oxidation of NACys into NDAC can lead to premature oxidation of NACys and overoxidation of NDAC into undesirable and difficult to remove sulfenic/sulfonic acids, sulfone and/or sulfoxide impurities. Therefore, a chemical process that avoids the use of thiol-containing raw materials and/or oxidizing conditions is highly desirable.

A sensible approach that avoids the use of thiol-containing raw materials with the potential to generate cystine derivatives like NDAC involves the use of cystine or cystine-derived raw materials. A practical process involving the direct conversion of cystine into NDAC has not been reported, mainly due to cystine's extremely low solubility in water and organic solvents (e.g. the solubility of cystine in water is 0.112 mg/ml at 25°C; cystine is more soluble in aqueous solutions with pH less than 2 or pH above 8; cystine is practically insoluble in organic solvents such as alcohols, ethers, esters, ketones, etc.). Alternative methods of NDAC synthesis starting with cystine derivatives would most likely entail protection-deprotection sequences. For instance, L-cystine diesters such as L-cystine dimethyl (DMC), diethyl (DEC) or di-tert-butyl (DTBC) esters are readily accessible as the dihydrochloride salts; these are also highly water soluble. Whereas N,N'-diacetylation of unhindered alkyl esters, such as DMC and DEC, under basic conditions to generate N,N'-dialkanoyl dialkylcystine esters is not practical due to partial disproportionation of DMC and DEC into by-products with undesirable sulfurous odor, hindered alkyl diesters like DTBC are more stable and attractive. Further, deprotection of tert-butyl ester functionalities under mild acidic conditions would generate N,N'-dialkanoyl cystines, NDAC being a representative within this class of compounds.

N,N'-dialkanoyl-di-tert-butyl-L-cystines, have been synthetically prepared directly from di-tert-butyl-L-cystine (1) and acid chlorides (see for example Liebigs Annalen der Chemie (1987) 895-9; Journal of Inorganic Biochemistry (2011) 105, 880-886), carboxylic acids (see for example Bioconjugate Chemistry (2004) 15, 541-553; Journal of the American Chemical Society (2001) 123, 1023-1035; Biochemistry (2005), 44, 9971-9979; US20170342046) or activated carboxylic acids (see for example WO2013002329). Also, a chemical process to prepare N,N'-diacetyl-di-tert-butyl-L-cystine (2) directly from di-tert-butyl-L-cystine (1) and acetic anhydride is described in European Journal of Organic Chemistry (2008) 26, 4417-4425. Unfortunately, these methods use undesirable organic solvents such as pyridine, chloroform, N,N-dimethylformamide, dichloromethane, acetonitrile and/or mixed solvents systems. Such organic solvents are toxic and/or flammable and impractical at the commercial scale, and in any event, require complex methods of purification such as acid-base extractions and chromatography. Further, some of these processes require long reaction times (e.g. 2 days) and yields that require additional purification steps to achieve the high purities (e.g. >99%) needed for ingredient qualification. Therefore, there is a need in the art for a practical and green chemical process to generate N,N'-diacetyl-di-tert-butyl-L-cystine (**2**) directly from di-tert-butyl-L-cystine (**1**), i.e., reagents with chemical structures shown below.

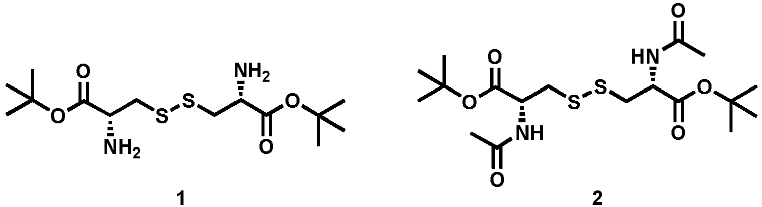

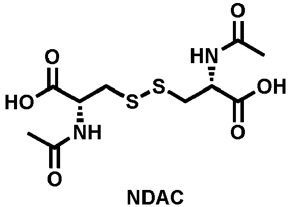

Direct deprotection of N,N'-dialkanoyl-di-tert-butyl-L-cystines to generate N,N'-dialkanoyl-cystines has been reported (see for example Journal of Inorganic Biochemistry (2011) 105, 880-886; WO2013002329; Journal of Enzyme Inhibition and Medicinal Chemistry (2018) 33, 1392-1404). Unfortunately, these methods use TFA in DCM or HCl in dioxane, which are not practical or green methods at the commercial scale. Therefore, there is a need in the art for a practical or green chemical process to specifically prepare N,N'-diacetyl-L-Cystine ("NDAC") directly from N,N'-diacetyl-di-tert-butyl-L-cystine (**2**).

Journal of Biological Chemistry (1931) 243-252 discloses the preparation of Diacetyl-L-cystine (NDAC) from cystine in water and with Ac2O as acetylation reagent. Purification is done using a mixture of acetone and water, in which acetylcystine is soluble.

### SUMMARY OF THE INVENTION

The present invention provides a practical two-step process to prepare N,N'-diacetyl-L-Cystine ("NDAC") from a cystine derivative.

Firstly, the present invention obviates the needs of the prior art by providing a chemical process to prepare N,N'-diacetyl-di-tert-butyl-L-cystine (**2**) using water as the solvent, takes less than 16 hours, does not need any extractive workup or purification, the product is isolated by simple filtration and the yields are high ( greater than 88%). Advantageously, the starting material is di-tert-butyl-L-cystine dihydrochloride, a commercially available white solid.

Additionally, the present invention obviates the needs of the prior art by providing a chemical process to prepare N,N'-diacetyl-L-Cystine ("NDAC") using formic acid as both the reagent and solvent and can be recycled, takes less than 3 hours, does not need any extractive workup or purification, the product is isolated upon solvent evaporation with no need for purification and the yields are high ( about 97%). Formic acid has significant advantages over other reagents used for this purpose (such as TFA and HCl) in that it is non-toxic, biodegradable and reusable (see Chinese Journal of Catalysis (2015) 36, 1461-1475).

The present invention includes a chemical process of making N-N'-diacetyl-L-cystine ("NDAC"), the process comprising the steps:
Forming a reaction mixture, starting with a cystine derivative di-tert-butyl-L-cystine ester (**1**) as the dihydrochloride form;,
Acetylating said di-tert-butyl-L-cystine to obtain N,N'-diacetyl-di-tert-butyl-L-cystine (**2**), followed by

Removing said tert-butyl groups from said N,N'-diacetyl-di-tert-butyl-L-cystine and isolating N,N'-diacetyl-L-Cystine ("NDAC") product; wherein said acetylating agent is acetic anhydride.

The inventive process is fast, uses green reagents (water and formic acid), can be carried out in a single reaction vessel, does not require labor-intensive isolation or purification of the product, NDAC, and has improved yield and purity over other reported methods. Further, the inventive process does not use thiol-containing cysteine derivatives or cystine derivatives that lead to by-products with undesirable sulfurous odor. A procedure is as follows:

### Step 1 - N,N'-diacetyl-di-tert-butyl-L-cystine (2)

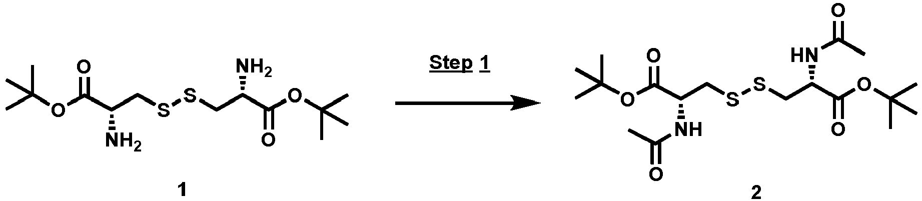

As a general procedure, Step 1 of the inventive process is preparation of N,N'-diacetyl-di-tert-butyl-L-cystine (**2**), an intermediate for NDAC production. Step 1 starts with preparing a solution of di-tert-butyl-L-cystine (**1**) (1 molar equivalent) from the dihydrochloride salt in water at temperatures ranging between 5-50 °C (preferably between 5 °C and 35 °C, most preferably between 10-25 °C).

Step 1 includes adding then acetylating agent acetic anhydride (2-5 molar equivalents) to the solution..

Step 1 further includes adding a base, preferably an inorganic base, (2-6 molar equivalents) to the solution, to form a mixture of di-tert-butyl-L-cystine (**1**), an acetylating agent, and a base. Inorganic base includes, for example, an alkali metal or alkaline earth metal bicarbonate or carbonate, such as sodium carbonate or sodium bicarbonate. Also suitable are an alkali metal hydroxide such as sodium hydroxide or an alkaline earth metal hydroxide or oxide such as calcium hydroxide or calcium oxide, and mixtures thereof. Preferred is sodium bicarbonate.

A reaction occurs by continuing to stir the mixture, at temperatures ranging between 5-50 °C (preferably between 5-35 °C, most preferably between 10-25 °C). Stirring continues until all the di-tert-butyl-L-cystine (starting material) is consumed (typically between 1-24h) and the desired N,N'-diacetyl-di-tert-butyl-L-cystine product precipitates out of solution.

The N,N'-diacetyl-di-tert-butyl-L-cystine **(2)** product (intermediate for NDAC) is obtained by filtering off and washing with water.

Step 2 of the inventive process is preparation of N,N'-diacetyl-L-Cystine, the final NDAC product.

Step 2 includes providing a suitable acid, including, but are not limited to, for example, formic, sulfuric, phosphoric or hydrochloric acid, and mixtures thereof (preferably formic acid due to its non-toxic, biodegradable and reusable properties and suitability as both a solvent and reagent). Suitable concentration is between 0.5ml-10ml acid per mmol of the N,N'-diacetyl-di-tert-butyl-L-cystine.

Step 2 includes suspending N,N'-diacetyl-di-tert-butyl-L-cystine (**2**) (1 molar equivalent) in the acid to form a suspension.

Step 2 includes stirring the suspension at temperatures ranging between 20 °C and 100 °C (preferably between 40-80 °C, most preferably between 50-70 °C) to generate a clear and colorless homogeneous solution. Stirring is continued until all the N,N'-diacetyl-di-tert-butyl-L-cystine (starting material for Step 2) is consumed (typically between 1-24h) and the desired product N,N'-diacetyl-L-Cystine ("NDAC") is generated.

N,N'-diacetyl-L-Cystine ("NDAC") is obtained, as an amorphous solid, by evaporating the solvents. The solvents can be re-cycled and reused indefinitely for Step 2.

Further, Step 2 may include purification and isolation of N,N'-diacetyl-L-cystine as a higher quality crystalline white solid, by adding a water-immiscible organic solvent such as for example ethyl acetate to the amorphous N,N'-diacetyl-L-Cystine ("NDAC"), stirring vigorously to effect precipitation/solidification into a white solid. The last step of the process, may include isolating the precipitated N,N'-diacetyl-L-Cystine ("NDAC") product, by centrifugation or filtering, preferably by filtering.

An example of a specific preferred reaction scheme is shown below.

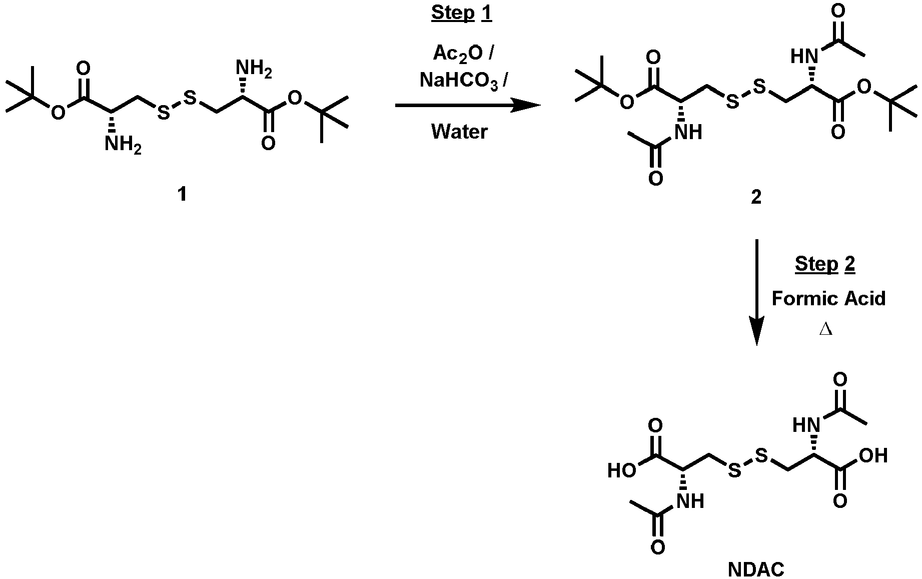

**Step 1** - **N,N'-diacetyl-di-tert-butyl-L-cystine (2)** - Acetic anhydride (0.55ml, 5.9mmol) was added to a solution of di-tert-butyl-L-cystine (1) dihydrochloride (1g, 2.4mmol) in water (10ml), followed by sodium bicarbonate (790mg, 9.4mmol) and the mixture stirred at room temperature (R.T.) for 16h at which point the product precipitated as a white solid. At this time, TLC (100% ethyl acetate elution) showed the clean formation of a major product. The product was filtered off and washed with water (3 X 10ml) and dried under vacuum to give pure N,N'-diacetyl-di-tert-butyl-L-cystine as a white solid which was pure enough for the next step (905mg, 88% yield).

**Step 2** - **N,N'-diacetyl-L-cystine (NDAC)** - N,N'-diacetyl-di-tert-butyl-L-cystine (2) (500mg, 1.15mmol) was suspended in formic acid (1ml) and heated at 60 °C for 3h to generate a clear and colorless homogeneous solution. At this time, TLC (30:40:30 ethyl acetate: isopropyl alcohol:water) showed the clean formation of a single product and no reactant/starting material. The solvents were removed under reduced pressure at 60 °C to give a colorless glassy gel. Optionally, the product was precipitated by adding ethyl acetate, filtered off and dried under high vacuum to give pure N,N'-diacetyl-L-cystine as a white solid (350mg, 97%).

In another preferred embodiment, the process is carried out in one overall step in a single vessel without isolation of intermediate (2). The process comprises adding all reagents, allowing step 1 to occur, adding step 2 reagent, heating, adding alcohol to precipitate salts, filtering off salts, and evaporating alcohol to isolate NDAC.

### DETAILED DESCRIPTION OF THE INVENTION

Except in the examples, or where otherwise explicitly indicated, all numbers in this description indicating amounts of material or conditions of reaction, physical properties of materials and/or use are to be understood as modified by the word "about."

It should be noted that in specifying any range of concentration or amount, any particular upper concentration can be associated with any particular lower concentration or amount.

For the avoidance of doubt, the word "comprising" is intended to mean "including" but not necessarily "consisting of" or "composed of." In other words, the listed steps or options need not be exhaustive.

The present invention provides a two-step process to prepare N,N'-diacetyl-L-Cystine ("NDAC") from a cystine derivative. In a general aspect, the process comprises the steps:
Starting with a cystine derivative di-tert-butyl-L-cystine ester (1) as the dihydrochloride form,
Acetylating to obtain N,N'-diacetyl-di-tert-butyl-L-cystine (2), followed by
Removing the tert-butyl groups and isolating N,N'-diacetyl-L-Cystine ("NDAC") product, wherein said acetylating agent is acetic anhydride.

Firstly, the present invention obviates the needs of the prior art by providing a chemical process to prepare N,N'-diacetyl-di-tert-butyl-L-cystine **(2)** using water as the solvent, takes less than 16 hours, does not need any extractive workup or purification, the product is isolated by simple filtration and the yields are high ( greater than 88%). The starting material is di-tert-butyl-L-cystine **(1),** a commercially available white solid.

Additionally, the present invention obviates the needs of the prior art by providing a chemical process to prepare N,N'-diacetyl-L-cystine (**NDAC**) using formic acid as both the reagent and solvent that can be recycled. The reaction takes less than three hours, does not need any extractive workup or purification, the product is isolated upon solvent evaporation with no need for purification, and the yields are high (about 97%).

In another aspect, the present invention includes a chemical process of making N,N'-diacetyl-L-Cystine ("NDAC"), the process comprising the steps:
Starting with a derivative of L-cystine, Di-tert-butyl Ester (**1**) as the dihydrochloride salt, which is commercially available;
Acetylating the precursor in aqueous medium in the presence of acetic anhydride and sodium bicarbonate;
Removing the tert-butyl groups using formic acid as both a reagent and a solvent under heating conditions;
Isolating N,N'-diacetyl-L-Cystine ("NDAC") product from the reaction mixture.
All amino acids were L stereo isomers. L-Cystine (>98%) was purchased from Sigma. N,N'-diacetyl-L-cystine (95%) was purchased from CombiBlocks. Di-tert-butyl-L-cystine dihydrochloride (DTBC; 98%) was purchased from Bachem.

### Di-tert-butyl-L-Cystine ("DTBC")

As a starting material for the inventive process, , Di-tert-butyl-L-cystine ester (**1** or "DTBC"), which is commercially available as the dihydrochloride form in large quantities as a white powder solid, is chosen. DTBC is itself a glutathione precursor. However, the increased reactivity and labile primary free amine functionality of DTBC over NDAC makes it a less suitable candidate for cosmetic use and more suitable as a reagent. This starting reagent is also referred to herein as di-tert-butyl-L-cystine (**1** or "DTBC") or di-tert-butyl-L-cystine dihydrochloride.

### N,N'-diacetyl-L-Cystine ("NDAC")

Applicants have discovered that N,N'-diacetyl-L-cystine (Also referred to as N,N'-diacetyl-L-cystine or NDAC) and NDAC salts, such as disodium salts, are useful amides in personal care compositions. NDAC structure is shown below (C₁₀H₁₆N₂O₆S₂):

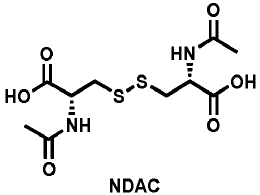

The molecular weight of NDAC = 324.4.

In skin cosmetic compositions, NDAC and its disodium salts, when applied to skin, are converted to cystine and cysteine intracellularly. Cysteine in turn has a number of cosmetic uses, including as a glutathione precursor. NDAC has superior functional benefits, including ultimate delivery of cysteine for cosmetic uses. Additionally, NDAC is more stable than simple alternative cystine esters such a DMC or DEC and does not result in unpleasant sulfur odor.

N,N'-diacetyl-L-Cystine ( "NDAC") may be prepared according to the inventive process.

According to another aspect, in the first step DTBC (**1**) is diacetylated so that N,N'-diacetyl-di-tert-butyl-L-cystine (**2**) is formed as described above. In the second step, N,N'-diacetyl-di-tert-butyl-L-cystine (**2**) is heated in the presence of formic acid which serves both as a solvent and reagent, thereby removing the tert-butyl group and resulting in NDAC product.

The inventive process is most useful for the synthesis of NDAC. As starting materials to prepare NDAC via its di-tert-butyl intermediate, DTBC (**1**) is used, which is a common and stable reagent available commercially in large quantities as the dihydrochloride salt. Upon reacting DTBC (**1**) with the acetylating agent acetic anhydride, N,N'-diacetyl-di-tert-butyl-L-cystine (**2**) is obtained. Next, N,N'-diacetyl-di-tert-butyl-L-cystine (**2**) is heated in the presence of formic acid which serves both as a solvent and reagent, thereby removing the tert-butyl group and resulting in NDAC product. The NDAC prepared according to the inventive process has superior functional benefits.

### Inventive Procedure

As a general procedure, Step 1 of the inventive process is preparation of N,N'-diacetyl-di-tert-butyl-L-cystine **(2),** an intermediate for NDAC production.

Step 1 starts with preparing a solution of di-tert-butyl-L-cystine (**1**) (1 molar equivalent) as the dihydrochloride salt in water at temperatures ranging between 5-50 °C (preferably between 5-35 °C, most preferably between 10-25 °C).

Step 1 includes adding the acetylating agent acetic anhydride (2-5 molar equivalents) to the solution..

Step 1 further includes adding an alkali metal or alkaline earth metal bicarbonate or carbonate (2-6 molar equivalents) to the solution, to form a mixture of di-tert-butyl-L-cystine (**1**), the acetylating agent, and alkali metal or alkaline earth metal bicarbonate or carbonate. Suitable are sodium bicarbonate or an alkali metal hydroxide such as sodium hydroxide (NaOH), or an alkaline earth metal hydroxide or oxide such as calcium hydroxide or calcium oxide. Preferred is sodium bicarbonate.

A reaction is allowed to occur by continuing to stir the mixture, at temperatures ranging between 5-50 °C (preferably between 5-35 °C, most preferably between 10-25 °C). Stirring continues until all the di-tert-butyl-L-cystine (starting material) is consumed (typically between 1-24h) and the desired N,N'-diacetyl-di-tert-butyl-L-cystine product precipitates out of solution.

The N,N'-diacetyl-di-tert-butyl-L-cystine (2) product (intermediate for NDAC) is filtered off and washed with water.

Step 2 of the inventive process is preparation of N,N'-diacetyl-L-Cystine ("NDAC") , the final NDAC product.

Step 2 includes providing a suitable acid including, but are not limited to, for example, formic, sulfuric, phosphoric or hydrochloric acid, and mixtures thereof (preferably formic acid due to its non-toxic, biodegradable and reusable properties and suitability as both a solvent and reagent). Suitable concentration is between 0.5ml-10ml acid per mmol of the N,N'-diacetyl-di-tert-butyl-L-cystine (**2**).

Step 2 includes suspending N,N'-diacetyl-di-tert-butyl-L-cystine (**2**) (1 molar equivalent) in the acid to form a suspension.

Step 2 includes stirring the suspension at temperatures ranging between 20-100 °C (preferably between 40-80 °C, most preferably between 50-70 °C) to generate a clear and colorless homogeneous solution. Stirring is continued until all the N,N'-diacetyl-di-tert-butyl-L-cystine (starting material for Step 2) is consumed (typically between 1-24h) and the desired product N,N'-diacetyl-L-Cystine ("NDAC") is generated.

The solvents are evaporated to give N,N'-diacetyl-L-Cystine ("NDAC") as an amorphous solid.

Further, Step 2 may include purification and isolation of N,N'-diacetyl-L-cystine as a higher quality crystalline white solid, by adding a water-immiscible organic solvent such as for example ethyl acetate to the amorphous N,N'-diacetyl-L-cystine, stirring vigorously to effect precipitation/solidification into a white solid. The last step of the process, may include isolating the precipitated N,N'-diacetyl-L-Cystine ("NDAC") product, by centrifugation or filtering, preferably by filtering.

All starting materials suitable for the inventive process are available from commercial sources, e.g. from Sigma-Aldrich, Bachem.

Particularly preferred in Step 1 is sodium bicarbonate, because it generates non-toxic sodium chloride as a by-product in solution which is easily separated from the product via filtration.

The relative amounts of the reagents and solvents are such as to not have excessive starting ingredients to minimize the amount of waste upon reaction completion.

Preferably the inventive process also comprises optional steps. For example optional step (1-A), wherein the water filtrate solution containing sodium acetate and sodium chloride by-products generated upon filtration of intermediate N,N'-diacetyl-di-tert-butyl-L-cystine (**2**) can be reused for additional cycles of step 1 or processed to recycle the water and independently isolate sodium acetate (or acetic acid) and sodium chloride for other uses. Optional step (2-A), wherein the formic acid used in step 2 is collected and recycled for additional cycles of step 2. Both of these optional steps allow for improved product turnover and reduced waste.

The inventive process is advantageous, at least because it does not use any toxic, hazardous or flammable organic solvents, results in the minimal formation of by-products, if any, does not generate product with undesirable sulfurous odor, both steps can be carried out in a single vessel and is relatively fast. It also results in improved purity of from 90% to 99.9%, preferably from 95 to 99.5%, and most preferably at least 95%, and improved overall yield from 85% to 95%, preferably from 90%, and most preferably at least 90% to 95%.

### Examples

### Experimental Methods

All reagents and solvents were obtained from commercial sources and used without further purification. All amino acids purchased were L stereo isomers. L-Cystine (>98%) was purchased from Sigma. N,N'-diacetyl-L-cystine (NDAC; 95%) was purchased from CombiBlocks. Di-tert-butyl-L-cystine dihydrochloride (DTBC; 98%) was purchased from Bachem.

Reaction monitoring was performed using thin layer chromatography (TLC) on silica gel using mixtures of ethyl acetate, isopropyl alcohol and water. Visualization of TLC plates was performed by subjecting TLC plates to 2% ninhydrin in ethanol followed by heat. Qualitative analysis and confirmation of reaction products was performed using 1H nuclear magnetic resonance (1H NMR) and liquid chromatography mass spectrometry (LCMS). Purity of reaction products was assessed via comparison of pure authentic standards using a combination of TLC, 1H NMR and LCMS methods.

### EXAMPLE 1

An example of the process within the scope of the invention was performed.

A specific nonexclusive representative experimental procedure was carried out in two steps as follows.

### Step 1 - N,N'-diacetyl-di-tert-butyl-L-cystine (2) -

Acetic anhydride (0.55ml, 5.9mmol) was added to a solution of di-tert-butyl-L-cystine (1) dihydrochloride (1g, 2.4mmol) in water (10ml), followed by sodium bicarbonate (790mg, 9.4mmol) and the mixture stirred @ R.T. for 16h at which point the product precipitated as a white solid.

At this time, TLC (100% ethyl acetate elution) showed the clean formation of a major product.

The product was filtered off and washed with water (3 X 10ml) and dried under vacuum to give pure N,N'-diacetyl-di-tert-butyl-L-cystine as a white solid which was pure enough for the next step (905mg, 88% yield). Other trials of this same process at various scales were completed in only 1.5 hours and gave yields of up to 95%. Product identity and purity (>95%) was confirmed by 1H NMR and LCMS.

### Step 2 - N,N'-diacetyl-L-cystine (NDAC) -

N,N'-diacetyl-di-tert-butyl-L-cystine (2) (500mg, 1.15mmol) was suspended in formic acid (1ml) and heated at 60 °C for 3h to generate a clear and colorless homogeneous solution.

At this time, TLC (30:40:30 ethyl acetate: isopropyl alcohol:water) showed the clean formation of a single product and no reactant/starting material.

The solvents were removed under reduced pressure at 60 °C to give a colorless glassy gel.

Optionally, the product was precipitated by adding ethyl acetate, filtered off and dried under high vacuum to give pure N,N'-diacetyl-L-Cystine ("NDAC") as a white solid (350mg, 97% yield). Acidic reagents other than formic acid (sulfuruc acid, acetic acid and Amberlyst^{®} 15 hydrogen form) were also tested and gave successful conversion of NDAC. Product identity and purity (>95%) was confirmed by 1H NMR and LCMS via comparison with an NDAC (95% purity) authentic standard.

The specific experimental procedure is summarized in the chemical diagram below:

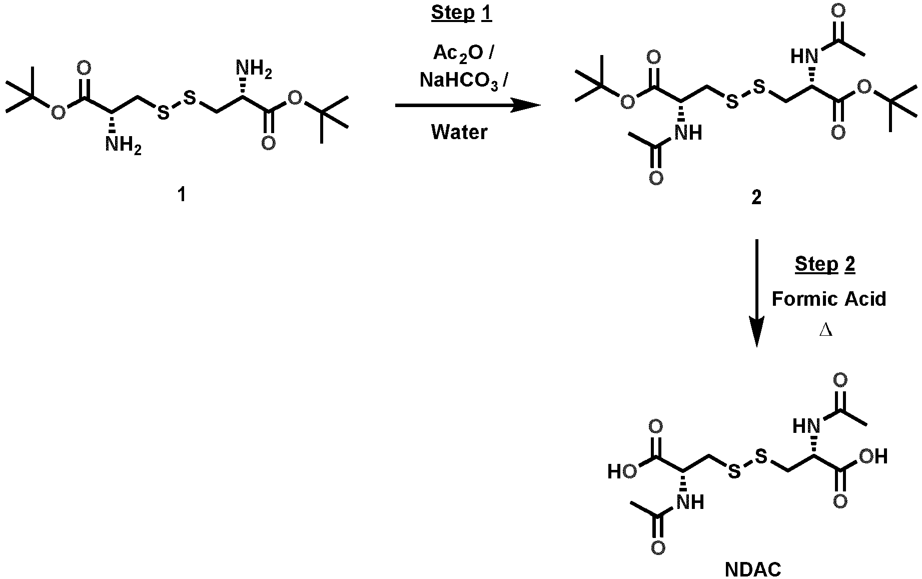

### EXAMPLE 2

An example of the process within the scope of the invention was performed.

A specific nonexclusive representative experimental procedure was carried out in one step in a single vessel as follows.

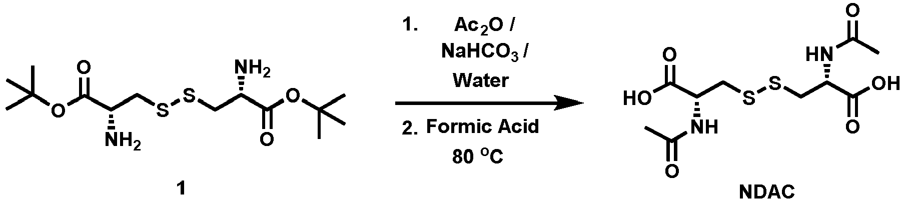

### N,N'-diacetyl-L-cystine (NDAC) -

Acetic anhydride (0.28ml, 2.9mmol) was added to a solution of di-tert-butyl-L-cystine (1) dihydrochloride (0.5g, 1.2mmol) in water (5ml), followed by sodium bicarbonate (395mg, 4.7mmol) and the mixture stirred @ R.T. for 1.5h at which point the product precipitated as a white solid.

At this time, TLC (100% ethyl acetate elution) showed the clean formation of intermediate N,N'-diacetyl-di-tert-butyl-L-cystine (2).

Formic acid (3ml) was added and heated at 80 °C for 5h to generate a clear and colorless homogeneous solution.

At this time, TLC (30:40:30 ethyl acetate: isopropyl alcohol:water) showed the clean formation of NDAC.

The solvents were removed under high vacuum at between 70-80 °C and the solid residue suspended in ethanol (6mL), stirred for 10min, filtered off and washed with ethanol (2 X 3ml).

The combined ethanol filtrates were evaporated at 50 °C under high vacuum to give pure NDAC as a white solid (270mg, 71% yield). Product identity and purity (>95%) was confirmed by TLC, 1H NMR and LCMS methods.

As demonstrated from the above procedure, the 2-step inventive process can be further simplified and conveniently carried out in a single vessel without the isolation of intermediate N,N'-diacetyl-di-tert-butyl-L-cystine (2). In this case isolation of the final product NDAC can be conveniently carried out by adding alcohol (to precipitate NaCl and sodium acetate salts) and simple filtration.

### COMPARATIVE EXAMPLE A

The following **out-of-scope example** illustrates **criticality of the acetylating reagent** under the inventive conditions. In this example acetyl chloride (an acid chloride) was used instead of acetic anhydride, resulting in no intermediate N,N'-diacetyl-di-tert-butyl-L-cystine product formation.

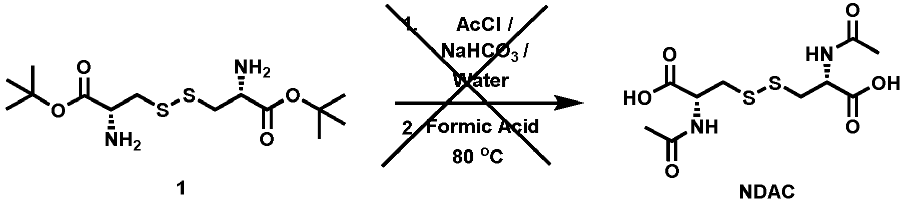

### COMPARATIVE EXAMPLE A

Sodium bicarbonate (158mg, 1.9mmol) was added to a solution of di-tert-butyl-L-cystine (1) dihydrochloride (0.2g, 0.5mmol) in water (2ml), followed by acetyl chloride (134uL, 1.9mmol) and the mixture stirred @ R.T. for 1.5h at which point the solution remained a clear and colorless homogeneous solution.

At this time, TLC (100% ethyl acetate elution) did not show the formation of intermediate N,N'-diacetyl-di-tert-butyl-L-cystine (2) and only di-tert-butyl-L-cystine (1) dihydrochloride starting material was present.

As demonstrated from the example above, the nature of the acetylating agent is absolutely critical for the success of the inventive process. Equally important, this out-of-scope example further illustrates the uniqueness of the inventive process, considering that acid chlorides have been used as acetylating reagents to prepare other N,N'-dialkanoyl-di-tert-butylcystines from di-tert-butylcystine (see for example Liebigs Annalen der Chemie (1987) 895-9; Journal of Inorganic Biochemistry (2011) 105, 880-886).

### COMPARATIVE EXAMPLE B

For comparison purposes, a process was carried out in accordance with European Journal of Organic Chemistry (2008) 26, 4417-4425.

### COMPARATIVE EXAMPLE B - N,N'-diacetyl-di-tert-butyl-L-cystine (2)

Acetic anhydride (2.7mL, 29mmol) was added to a suspension of di-tert-butyl-L-cystine dihydrochloride (2.48g, 5.83mmol) in pyridine (25mL; anhydrous) and cooled to 0 °C. After stirring the mixture to generate a homogeneous solution and allowing the exothermic reaction to subdue, the reaction mixture was placed at 4 °C for 2d. Ice was added, followed by portionwise addition of hydrochloride acid (12.1M, 25 mL) to maintain the temp below 20°C and the pH between 4-5. The reaction mixture was extracted with ether (4 X 100mL) and the combined organic layers washed with aqueous sulfuric acid (0.5 M) until pH between 2-3, followed by saturated sodium bicarbonate. After drying the organic layer and removing the solvent, the residue was dissolved in chloroform (20 mL) and hexane added until cloudiness persisted. The product was allowed to crystallize at 4 °C overnight, filtered and dried to give 2.16 g (85%).

As can be seen from the procedure above, the non-aqueous process to generate N,N'-diacetyl-di-tert-butyl-L-cystine (2) intermediate took much longer (3 days total) than the inventive process (1.5 to 16 hours), involved the use of toxic and flammable organic solvents (pyridine, ether, chloroform and hexane) versus the inventive process (water), resulted in slightly decreased yield (85%) versus the inventive process (88 to 95%) and was harder to purify (extractive work-up and crystallization) versus inventive process (filtration and water washing).

## Claims

1. A chemical process of making N-N'-diacetyl-cystine ("NDAC"), the process comprising the steps:
Forming a reaction mixture, starting with a cystine derivative di-tert-butyl-L-cystine as the dihydrochloride form;
Acetylating said di-tert-butyl-L-cystine to obtain N,N'-diacetyl-di-tert-butyl-L-cystine ; followed by
Removing said tert-butyl groups from said N,N'-diacetyl-di-tert-butyl-L-cystine to obtain N,N'-diacetyl-L-cystine product; and
Isolating said N,N'-diacetyl-L-Cystine product from said reaction mixture; wherein said acetylating agent is acetic anhydride.

2. A process of making N,N'-diacetyl-L-Cystine according to claim 1 , the process comprising the steps:
(1) preparing N,N'-diacetyl-di-tert-butyl-L-cystine as an intermediate, by:
preparing an aqueous 1 molar equivalent solution of di-tert-butyl-L-cystine as the dihydrochloride form, at temperature of 5 to 50°C, preferably from 5 to 35°C, most preferably from 10 to 25 °C;
adding from 2 to 5 molar equivalents of an acetylating agent to the solution; wherein said acetylating agent is acetic anhydride;adding to said solution from 2 to 6 molar equivalents of a base, to form a mixture of di-tert-butyl-L-cystine, the acetylating agent, and a base;
Stirring said mixture continually and allowing a reaction to occur, at temperature from 5 to 50 °C, preferably from 5 to 35 °C, most preferably from 10 to 25 °C, to precipitate N,N'-diacetyl-di-tert-butyl-L-cystine intermediate product out of the reaction mixture;
Filtering off N,N'-diacetyl-di-tert-butyl-L-cystine and washing with water;
(2) preparing N,N'-diacetyl-L-Cystine product by:
providing from between 0.5ml-10ml acid per mmol of the N,N'-diacetyl-di-tert-butyl-L-cystine including,
suspending 1 molar equivalent N,N'-diacetyl-di-tert-butyl-L-cystine in said acid to form a suspension;
stirring said suspension, at a temperature from 20 to 100 °C preferably from 40 to 80 °C, most preferably from 50 to 70 °C, to generate a clear and colorless homogeneous solution;
Evaporating the solution solvents to give N,N'-diacetyl-L-Cystine as an amorphous solid.

3. The process according to claim 2, wherein the base is an inorganic base.

4. The process according to any one of claims 2-3, wherein the base is selected from the group consisting of (i) an alkali metal bicarbonate or carbonate, (ii) alkaline earth metal bicarbonate or carbonate, (iv) alkali metal hydroxide, and (iii) mixtures thereof.

5. The process according to any one of claims 2-3, wherein said base is selected from the group consisting of sodium bicarbonate, sodium carbonate, sodium hydroxide, calcium hydroxide, calcium oxide, and mixtures thereof; preferably sodium bicarbonate.

6. The process according to claim 2, step (1), wherein said stirring of said basic mixture continues until all the di-tert-butyl-L-cystine (starting material) is consumed, for from 1 to 24h, and until N,N'-diacetyl-di-tert-butyl-L-cystine product precipitates out of solution.

7. The process according to claim 2, wherein said acid is selected from the group consisting of formic, sulfuric, phosphoric or hydrochloric acid, and mixtures thereof; preferably formic acid.

8. The process according to claim 2, step (2), wherein said stirring of said acid mixture continues until all the all the N,N'-diacetyl-di-tert-butyl-L-cystine is consumed, for from 1 to 24h, and the desired product N,N'-diacetyl-L-cystine is generated.

9. The process according to any one of claims 2-8, further comprising purifying and isolating said N,N'-diacetyl-L-cystine as a higher quality crystalline white solid, by adding a water-immiscible organic solvent to the amorphous N,N'-diacetyl-L-Cystine ; and stirring vigorously to effect precipitation/solidification into a white solid.

10. The process according to claim 9, wherein said organic solvent is ethyl acetate.

11. The process according to claim 9 or 10, Further comprising a last step comprising isolating the precipitated N,N'-diacetyl-L-cystine product, by centrifugation or filtration, preferably by filtration.

12. The process according to any of the preceding claims, wherein the reaction is carried out in one step in a single vessel without isolation of intermediate (2).

13. The process according to any of the preceding claims, comprising:
adding all reagents, allowing step 1 to occur, adding step 2 reagent, heating, adding alcohol to precipitate salts, filtering off salts, and evaporating alcohol to isolate NDAC.

## Patentansprüche

1. Chemisches Verfahren zur Herstellung von N,N'-Diacetyl-cystin ("NDAC"), wobei das Verfahren die Schritte umfasst:
Bilden einer Reaktionsmischung, beginnend mit einem Cystinderivat Di-tert-butyl-L-cystin als Dihydrochloridform;
Acetylieren des Di-tert-butyl-L-cystins, um N,N'-Diacetyl-di-tert-butyl-L-cystin zu erhalten; gefolgt vom
Entfernen der tert-Butylgruppen von dem N,N'-Diacetyl-di-tert-butyl-L-cystin, um das N,N'-Diacetyl-L-cystin-Produkt zu erhalten; und
Isolieren des N,N'-Diacetyl-L-cystin-Produkts aus der Reaktionsmischung, wobei das Acetylierungsmittel Essigsäureanhydrid ist.

2. Verfahren zur Herstellung von N,N'-Diacetyl-L-cystin nach Anspruch 1, wobei das Verfahren die Schritte umfasst:
(1) Herstellen von N,N'-Diacetyl-di-tert-butyl-L-cystin als Zwischenprodukt durch:
Herstellen einer wässrigen 1-mol-äquivalenten Lösung von Di-tert-butyl-L-cystin als Dihydrochloridform bei einer Temperatur von 5 bis 50°C, vorzugsweise von 5 bis 35°C, höchst bevorzugt von 10 bis 25°C;
Zugeben von 2 bis 5 Moläquivalenten eines Acetylierungsmittels zu der Lösung, wobei das Acetylierungsmittel Essigsäureanhydrid ist; Zugeben von 2 bis 6 Moläquivalenten einer Base zu der Lösung, um eine Mischung des Di-tert-butyl-L-cystins, des Acetylierungsmittels und einer Base zu bilden;
kontinuierliches Rühren der Mischung und Ablaufenlassen einer Reaktion bei einer Temperatur von 5 bis 50°C, vorzugsweise von 5 bis 35°C, höchst bevorzugt von 10 bis 25°C, um das N,N'-Diacetyl-di-tert-butyl-L-cystin-Zwischenprodukt aus der Reaktionsmischung auszufällen;
Abfiltrieren von N,N'-Diacetyl-di-tert-butyl-L-cystin und Waschen mit Wasser;
(2) Herstellen des N,N'-Diacetyl-L-cystin-Produkts durch:
Bereitstellen von 0,5 ml - 10 ml Säure pro mmol des N,N'-Diacetyl-di-tert-butyl-L-cystins, einschließlich
Suspendieren von 1 Moläquivalent N,N'-Diacetyl-di-tert-butyl-L-cystin in Säure, um eine Suspension zu bilden;
Rühren der Suspension bei einer Temperatur von 20 bis 100°C, vorzugsweise von 40 bis 80°C, höchst bevorzugt von 50 bis 70°C, um eine klare und farblose homogene Lösung zu erzeugen;
Verdampfen der Lösungsmittel der Lösung, um N,N'-Diacetyl-L-cystin als amorphen Feststoff zu erhalten.

3. Verfahren nach Anspruch 2, wobei die Base eine anorganische Base ist.

4. Verfahren nach irgendeinem der Ansprüche 2-3, wobei die Base aus der Gruppe ausgewählt ist, bestehend aus (i) einem Alkalimetallbicarbonat oder - carbonat, (ii) einem Erdalkalimetallbicarbonat oder -carbonat, (iv) einem Alkalimetallhydroxid und (iii) Mischungen davon.

5. Verfahren nach irgendeinem der Ansprüche 2-3, wobei die Base aus der Gruppe ausgewählt ist, bestehend aus Natriumbicarbonat, Natriumcarbonat, Natriumhydroxid, Calciumhydroxid, Calciumoxid und Mischungen davon, vorzugsweise Natriumbicarbonat.

6. Verfahren nach Anspruch 2, Schritt (1), wobei das Rühren der basischen Mischung 1 bis 24 h lang fortgesetzt wird, bis das gesamte Di-tert-butyl-L-cystin (Ausgangsmaterial) verbraucht ist und bis das N,N'-Diacetyl-di-tert-butyl-L-cystin-Produkt aus der Lösung ausfällt.

7. Verfahren nach Anspruch 2, wobei die Säure aus der Gruppe ausgewählt ist, bestehend aus Ameisensäure, Schwefelsäure, Phosphorsäure oder Salzsäure und Mischungen davon, vorzugsweise Ameisensäure.

8. Verfahren nach Anspruch 2, Schritt (2), wobei das Rühren der sauren Mischung 1 bis 24 h lang fortgeführt wird, bis das gesamte N,N'-Diacetyl-di-tert-butyl-L-cystin verbraucht ist und das gewünschte Produkt N,N'-Diacetyl-L-cystin erzeugt ist.

9. Verfahren nach irgendeinem der Ansprüche 2-8, ferner umfassend das Reinigen und Isolieren des N,N'-Diacetyl-L-cystins als einen kristallinen weißen Feststoff hoher Qualität durch Zugabe eines mit Wasser nicht mischbaren organischen Lösungsmittels zu dem amorphen N,N'-Diacetyl-L-cystin und heftiges Rühren, um eine Ausfällung/Verfestigung zu einem weißen Feststoff zu bewirken.

10. Verfahren nach Anspruch 9, wobei das organische Lösungsmittel Ethylacetat ist.

11. Verfahren nach Anspruch 9 oder 10, ferner umfassend einen letzten Schritt, der die Isolierung des ausgefällten N,N'-Diacetyl-L-cystin-Produkts durch Zentrifugieren oder Filtrieren, vorzugsweise durch Filtrieren, umfasst.

12. Verfahren nach einem der vorhergehenden Ansprüche, wobei die Reaktion in einem Schritt in einem einzelnen Behälter ohne Isolieren des Zwischenprodukts (2) durchgeführt wird.

13. Verfahren nach einem der vorhergehenden Ansprüche, umfassend: Zugeben aller Reagenzien, Ablaufenlassen von Schritt 1, Zugeben des Schritt 2-Reagenzes, Erhitzen, Zugeben von Alkohol zum Ausfällen von Salzen, Abfiltrieren von Salzen und Verdampfen von Alkohol zum Isolieren von NDAC.

## Revendications

1. Procédé chimique de production de N-N'-diacétyl-cystine ("NDAC"), le procédé comprenant les étapes de :
formation d'un mélange réactionnel, à partir du dérivé de cystine di-tert-butyl-L-cystine sous la forme du dichlorhydrate ;
acétylation de ladite di-tert-butyl-L-cystine pour que soit obtenue de la N,N'-diacétyl-di-tert-butyl-L-cystine ; suivie d'une
élimination desdits groupes tert-butyle sur ladite N,N'-diacétyl-di-tert-butyl-L-cystine pour que soit obtenu le produit N,N'-diacétyl-L-cystine ; et
isolation dudit produit N,N'-diacétyl-L-cystine à partir dudit mélange réactionnel ;
dans lequel ledit agent d'acétylation est l'anhydride acétique.

2. Procédé de production de N,N'-diacétyl-cystine selon la revendication 1, le procédé comprenant les étapes de :
(1) préparation de N,N'-diacétyl-di-tert-butyl-L-cystine en tant qu'intermédiaire, par :
préparation d'une solution aqueuse de 1 équivalent molaire de di-tert-butyl-L-cystine sous la forme du dichlorhydrate, à une température de 5 à 50°C, de préférence de 5 à 35°C, tout spécialement de 10 à 25°C ;
addition de 2 à 5 équivalents molaires d'un agent d'acétylation à la solution ; dans laquelle ledit agent d'acétylation est l'anhydride acétique ; addition à ladite solution de 2 à 6 équivalents molaires d'une base, pour former un mélange de di-tert-butyl-L-cystine, de l'agent d'acétylation, et d'une base ;
agitation dudit mélange en continu, une réaction étant laissée à se dérouler, à une température de 5 à 50°C, de préférence de 5 à 35°C, tout spécialement de 10 à 25°C, pour précipiter le produit intermédiaire N,N'-diacétyl-di-tert-butyl-L-cystine à partir du mélange réactionnel ;
retrait par filtration de la N,N'-diacétyl-di-tert-butyl-L-cystine et lavage à l'eau ;
(2) préparation du produit N,N'-diacétyl-L-cystine par :
fourniture d'entre 0,5 ml et 10 ml d'acide par mmol de la N,N'-diacétyl-di-tert-butyl-L-cystine, y compris
mise en suspension de 1 équivalent molaire de N,N'-diacétyl-di-tert-butyl-L-cystine dans ledit acide pour former une suspension ;
agitation de ladite suspension, à une température de 20 à 100°C, de préférence de 40 à 80°C, tout spécialement de 50 à 70°C, pour générer une solution homogène limpide et incolore ;
évaporation des solvants de la solution pour donner la N,N'-diacétyl-L-cystine sous la forme d'un solide amorphe.

3. Procédé selon la revendication 2, dans lequel la base est une base inorganique.

4. Procédé selon l'une quelconque des revendications 2 et 3, dans lequel la base est choisie dans le groupe constitué par (i) un carbonate ou bicarbonate de métal alcalin, (ii) un carbonate ou bicarbonate de métal alcalino-terreux, (iv) un hydroxyde de métal alcalin, et (iii) leurs mélanges.

5. Procédé selon l'une quelconque des revendications 2 et 3, dans lequel ladite base est choisie dans le groupe constitué par le bicarbonate de sodium, le carbonate de sodium, l'hydroxyde de sodium, l'hydroxyde de calcium, l'oxyde de calcium, et leurs mélanges ; de préférence le bicarbonate de sodium.

6. Procédé selon la revendication 2, étape (1), dans lequel ladite agitation dudit mélange basique se poursuit jusqu'à ce que toute la di-tert-butyl-L-cystine (matériau de départ) soit consommée, ou pendant 1 à 24 heures, et jusqu'à ce que le produit N,N'-diacétyl-di-tert-butyl-L-cystine précipite à partir de la solution.

7. Procédé selon la revendication 2, dans lequel ledit acide est choisi dans le groupe constitué par les acides formique, sulfurique, phosphorique et chlorhydrique, et leurs mélanges ; de préférence l'acide formique.

8. Procédé selon la revendication 2, étape (2), dans lequel ladite agitation dudit mélange acide se poursuit jusqu'à ce que toute la N,N'-diacétyl-di-tert-butyl-L-cystine soit consommée, ou pendant 1 à 24 heures, et le produit souhaité N,N'-diacétyl-L-cystine est généré.

9. Procédé selon l'une quelconque des revendications 2 à 8, comprenant en outre la purification et l'isolation de ladite N,N'-diacétyl-L-cystine sous la forme d'un solide cristallin de qualité supérieure, par addition d'un solvant organique non miscible avec l'eau à la N,N'-diacétyl-L-cystine amorphe ; et agitation vigoureuse pour effectuer une précipitation/ solidification en un solide blanc.

10. Procédé selon la revendication 9, dans lequel ledit solvant organique est l'acétate d'éthyle.

11. Procédé selon la revendication 9 ou 10, comprenant en outre une dernière étape comprenant l'isolation du produit N,N'-diacétyl-L-cystine précipité, par centrifugation ou filtration, de préférence par filtration.

12. Procédé selon l'une quelconque des revendications précédentes, dans lequel la réaction est effectuée en une seule étape dans un seul récipient sans isolation d'intermédiaire (2).

13. Procédé selon l'une quelconque des revendications précédentes, comprenant : l'addition de tous les réactifs, le fait de laisser l'étape 1 se dérouler, l'addition du réactif de l'étape 2, le chauffage, l'addition d'alcool pour précipiter les sels, le retrait des sels par filtration, et l'évaporation de l'alcool pour isoler la NDAC.
